(19) Europäisches Patentamt
European Patent Office
Office européen des brevets

(11) **EP 4 599 779 A1**

(12) **EUROPEAN PATENT APPLICATION**

(43) Date of publication:
**13.08.2025 Bulletin 2025/33**

(21) Application number: **25155952.2**

(22) Date of filing: **05.02.2025**

(51) International Patent Classification (IPC):
**A61B 17/62** *(2006.01)* **A61B 17/64** *(2006.01)*
**A61B 90/00** *(2016.01)*

(52) Cooperative Patent Classification (CPC):
**A61B 17/62; A61B 17/645; A61B 90/39;**
A61B 17/6491; A61B 2090/3966

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **12.02.2024 US 202463552218 P**

(71) Applicant: **Stryker European Operations Limited Carrigtwohill, Co. Cork T45 HX08 (IE)**

(72) Inventors:
• **KUMAR, Anup**
**122002 Gurgaon, Haryana (IN)**
• **SINGHAL, Soniya**
**243005 Bareilly, Uttar Pradesh (IN)**
• **GUPTA, Anmol**
**560066 Bengaluru, Karnataka (IN)**
• **SAHA, Indrajit**
**400055 Mumbai, Maharashtra (IN)**
• **ALI, Rustam**
**827012 Bokaro Steel City, Jharkhand (IN)**
• **BHATIA, Purvi**
**121006 Faridabad, Haryana (IN)**
• **LEHMANN, Philippe**
**2516 Lamboing (CH)**

(74) Representative: **Schott, Jakob Valentin Wuesthoff & Wuesthoff Patentanwälte und Rechtsanwalt PartG mbB Schweigerstraße 2 81541 München (DE)**

(54) **REFERENCE DEVICES FOR EXTERNAL FIXATION FRAMES**

(57) A reference assembly connected to a support ring of an external fixation frame. The reference assembly includes a mount device. The mount device includes a mount body, a static member, and a dynamic member. The static member is fixedly secured to and extends from the mount body and is configured to be received within a first hole of the support ring of the external fixation frame. The dynamic member is slidably connected to the mount body and is configured to be received within a second hole of the support ring. The reference assembly also includes a reference device that is connectable to the mount body. The reference device includes reference body and a plurality of reference objects disposed within the reference body. The reference objects are each made from a radiopaque material, and the reference body is made from a radiolucent material.

FIG. 3B

EP 4 599 779 A1

**Description**

BACKGROUND

Various orthopedic procedures, such as bone deformity correction, fracture reduction, limb lengthening, and the like, often require the implementation of an external fixation frame. An external fixation frame is typically positioned over the target limb, secured to underlying bone segments, and incrementally adjusted for manipulation, lengthening, angulation, rotation, and/or translation of the bone segments. Such adjustments need to be made precisely and are often made on a frequent basis *(e.g.,* daily or weekly) during the patient's recovery.

Radiographic images are often utilized to determine proper placement and orientation of an external fixation frame relative to the bone, to develop a correction plan for future frame adjustments, and to ensure the adjustments being made are adequate. For example, two-dimensional medial-lateral ("M-L") and anterior-posterior ("A-P") radiographic images may be obtained to help determine the positioning of various fixation frame components (*e.g.*, support rings) relative to bone segments and relative to each other. From there, a correction plan may be developed instructing the various incremental adjustments that need to be made to the external fixation frame's components to achieve the desired clinical results. However, during this process, many sources of error may be introduced, not least of which being the use of two-dimensional image inputs of indeterminate scale and orientation to obtain precise three-dimensional outputs. Therefore, further improvements are desirable.

BRIEF SUMMARY OF THE DISCLOSURE

In one aspect of the present disclosure, a reference assembly may be connected to a support ring of an external fixation frame. The reference assembly may include a mount device that may have a mount body, a static member, and a dynamic member. The static member may be fixedly secured to and may extend from the mount body and may be configured to be received within a first hole of the support ring of the external fixation frame. The dynamic member may be slidably connected to the mount body and may be configured to be received within a second hole of the support ring. The reference assembly may also include a reference device that may be connectable to the mount body. The reference device may have a reference body and a plurality of reference objects disposed within the reference body. The reference objects may each be made from a radiopaque material, and the reference body may be made from a radiolucent material.

Additionally, the reference objects may include a first reference object that may have a first cross-sectional dimension, and a second reference object that may have a second cross-sectional dimension. The first cross-sectional dimension being greater than the second cross-sectional dimension. The reference objects may each be spherical.

Furthermore, the reference body may include a base and a plurality of reference columns extending from the base. Each reference column may include at least a first reference object and a second reference object. The first reference object may have a larger cross-sectional dimension than the second reference object. The first reference object of each reference column may also be positioned further from the base than the second reference object. Each reference column may further include a third and fourth reference object. The third and fourth reference objects may each have a cross-sectional dimension equal to the cross-sectional dimension of the second reference object. The first, second, third, and fourth reference objects of each reference column may each be spherical. The reference body may include four reference columns, and the reference objects of each reference column may be arranged colinearly. Further, the first reference objects of the reference columns may be coplanar, and the fourth reference objects of the reference columns may also be coplanar. However, the second reference objects of the reference columns may be longitudinally staggered relative to one another.

In addition, each reference column may include a longitudinal opening and a reference insert removably disposed therein. The reference insert may include the first and second reference objects. The longitudinal opening of each reference column may include a threaded portion, and each reference column may include a threaded portion correspondingly engageable with the threaded portion of the longitudinal opening of the respective reference column.

Continuing with this aspect, the mount body may include a slot, and the dynamic member may be slidably disposed within the slot. The slot may be at least partially defined by opposing rails, and the dynamic member may include a head having a first head portion, a second head portion, and a circumferential groove disposed between the first head portion and the second head portion. The rails may be disposed within the groove of the head when the dynamic member is received within the slot. Also, the mount body may include a chamfer surface that at least partially defines the slot, and the head may include a corresponding chamfer surface that engages the chamfer surface of the mount body.

In some examples, the dynamic member may include a threaded shaft. In other examples, the dynamic member may have a first shaft portion, a second shaft portion, and an O-ring disposed between the first shaft portion and the second shaft portion. In further examples, the dynamic member may include a radially expandable shaft. Such radially expandable shaft may include a plurality of legs which may have a first and second configuration. In the first configuration, the plurality of legs may be in a relaxed state, and, in the second configuration, the plurality of legs may be in an expanded state. The radially expandable shaft may include a plunger at least partially disposed within a cavity defined by the plurality of legs. The plunger may be axially moveable from a first position in which the legs may be in the relaxed state to a second position in which the plunger pushes the legs radially outwardly to the expanded state. The plunger may include a threaded shaft, and the dynamic member may include a head threadedly connected to the threaded shaft such that rotating the head in a first direction may move the plunger from the first position to the second position, and rotating the head in a second direction may move the plunger from the second position back to the first position.

Furthermore, the reference device may include a reference body and a connection member that may extend from the reference body. The connection member may define a recess configured to receive an end of the mount body. The reference assembly may also include a connector that may have a threaded shaft. The connection member may include a through-hole, and the reference body may include a threaded opening. The through-hole and the threaded opening may be configured to receive the threaded shaft of the connector to secure the reference device to the mount device. The recess may be defined at least by a pair of chamfer surfaces of the connection member, and the mount body may include a corresponding pair of chamfer surfaces configured to engage the chamfer surfaces of the connection member. The threaded opening of the mount body may be disposed between the pair of chamfer surfaces thereof.

In a further aspect of the present disclosure, an external fixation frame may include first and second support rings. The first support ring may have a first inner row of holes and a second outer row of holes. The external fixation frame may also include one or more struts connected to and extending between the first and second support rings. Each strut may have an adjustable length. Additionally, the external fixation frame may include a reference assembly that may include a mount device and a reference device. The mount device may have a mount body, a static member, and a dynamic member. The static member may be receivable within a hole of the second outer row of holes. The dynamic member may be moveable relative to the mount body and may be receivable within a hole of the first inner row of holes. The reference device may be connectable to the mount body and may have a plurality of reference objects that may each be radiopaque.

In a further aspect of the present disclosure, an external fixation frame may include first and second support rings. The first support ring may have a first inner row of holes and a second outer row of holes. The external fixation frame may also include one or more struts connected to and extending between the first and second support rings. Each strut may have an adjustable length. Additionally, the external fixation frame may include a reference assembly that may include a mount device and a reference device. The mount device may be connectable to the first inner row of holes and second outer row of holes. The reference device may be connectable to the mount body and may have a plurality of reference columns. Each reference column may have a first reference object and a second reference object. The first reference object may have a cross-sectional dimension greater than that of the second reference object, and the first and second reference objects may be radiopaque.

BRIEF DESCRIPTION OF THE DRAWINGS

FIG. 1 is a perspective view of an external fixation system according to an embodiment of the disclosure.
FIG. 2 is a perspective view of a reference assembly according to an embodiment of the disclosure and mounted to a support ring of the external fixation system of FIG. 1.
FIG. 3A is a perspective view of a reference device of the reference assembly of FIG. 2 according to an embodiment of the present disclosure.
FIG. 3B is a transparent perspective view of the reference device of FIG. 3A.
FIG. 3C is another perspective view of the reference device of FIG. 3A.
FIG. 3D is schematic two-dimensional view of a reference object pattern of the reference device of FIG. 3A.
FIG. 4A is perspective view of a connection device of the reference assembly of FIG. 3A according to an embodiment of the disclosure.
FIG. 4B is a cross-sectional view of the connection device of FIG. 4A taken along a midline of a dynamic member thereof.
FIG. 5A is an exploded view of FIG. 2.
FIG. 5B is a partial transparent bottom view of the reference assembly of FIG. 3A.
FIG. 5C is a partial bottom cross-sectional view of the reference assembly of FIG. 3A taken through a mount body, a fastener, and a connection member of a reference body thereof.
FIG. 5D is a partial cross-sectional view of the reference assembly of FIG. 3A taken through the mount body, the fastener, and the connection member of the reference body and orthogonal to the cross-sectional view of FIG. 5C.
FIG. 6A is a partial perspective view of a connection device according to another embodiment of the disclosure.
FIG. 6B is a cross-sectional view of the connection device of FIG. 6A taken along a midline of a dynamic member thereof.
FIG. 7 is an elevational view of a connection device according to a further embodiment of the disclosure.

FIG. 8 is an exemplary configuration of a computing device which may be used to implement aspects of the disclosure.
FIG. 9 is a flow diagram describing an example of a method that may be used to implement aspects of the disclosure.

DETAILED DESCRIPTION

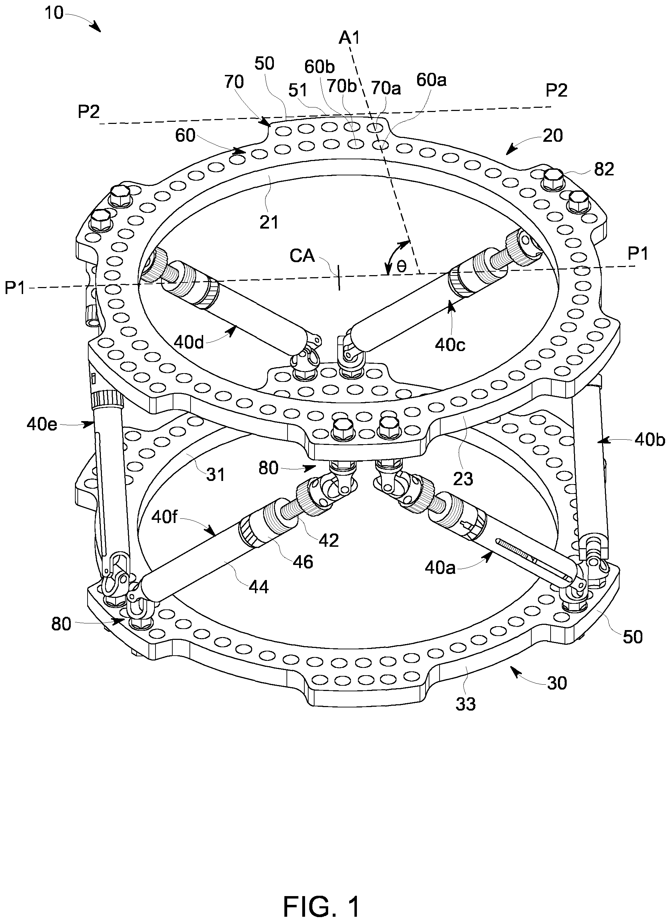

FIG. 1 depicts an external fixation frame 10 in an assembled condition according to one aspect of the disclosure. Generally, fixation frame 10 includes a first support ring 20 and a second support ring 30, with a plurality of adjustable length telescopic struts 40 coupling the first rings 20 to the second ring 30. Fixation frame 10 may be a hexapod frame such that it includes six telescopic struts 40, such as the depicted struts 40a-f. However, some frames 10 may have less struts 40, such as four struts 40, for example.

First ring 20 may also be referred to as a proximal ring or a reference ring, while second ring 30 may also be referred to as a distal ring or a moving ring. First and second rings 20, 30 each include an inner edge 21, 31, outer edge 23, 33, and a ring-diameter which extends between the outer edge 23, 33 at two locations thereof and through a center axis CA of the ring 20, 30. In the depicted example of fixation frame 10, first ring 20 and second ring 30 have the same ring-diameter. However, in some fixation frame examples, the ring diameters may differ.

First ring 20 and/or second ring 30 may include a plurality of extension tabs 50. In the illustrated example, each ring 20 and 30 includes six extension tabs 50 spaced circumferentially around a perimeter thereof and extending radially outwardly from the outer edge 23, 33. Although rings 20 and 30 are depicted as having six extension tabs 50, each ring 20, 30 may have more or less extension tabs 50 *(e.g.,* three tabs) depending on the particular components of fixation system 10 and/or the ring diameter. In addition to what is described directly below, extension tabs 50 may help increase the cross-sectional area of rings 20, 30 and thus provide for increased stiffness of rings 20, 30.

With this configuration, each ring 20, 30 may include a first inner circumferential row of holes 60 and a second outer circumferential row of holes 70. As illustrated, the second outer circumferential row of holes 70 may include at least a first hole 70a and a second hole 70b and may only be positioned on the plurality of extension tabs 50 on the rings 20 and 30. It should be understood that although the second outer circumferential row of holes 70 is shown in FIG. 1 as being positioned solely on extension tabs 50, top ring 20 and/or bottom ring 30 may contain two complete rows of holes, for example with a completely circular (or nearly completely circular) geometry. The use of extension tabs 50, compared to two full circumferential rows of holes, may help reduce overall bulk of rings 20, 30 and also provide for intuitive strut placement for surgical personnel. The completely circular version of rings 20, 30 with two full (or nearly full) rows of circumferential holes may be particularly suited for relatively small diameter rings, although indentations or other features may be introduced to provide an intuitive interface for strut placement by surgical personnel.

Further, in the illustrated embodiment, the first and second circumferential rows of holes 60 and 70 may be positioned so that the first row of holes 60 does not align radially with the second row of holes 70. In other words, first and second row of holes 60, 70 form hole pairs that may each have a staggered configuration relative to the central axis CA of ring 20, 30. For example, a first hole pair comprising a first hole 60a of the first row of holes 60 and a second hole 70a of the second row of holes 70 may be aligned along an axis A1 that intersects a first plane P1 containing central axis CA of first ring 20 at an oblique angle Θ and at a location offset from central axis CA, as shown in FIG. 1. For some hole pairs, the oblique angle Θ may be 45 degrees, for example. Also, as shown, the first plane P1 may be parallel with a second plane P2 that may be tangent with an apex or midpoint 51 of tab 50. Thus, the staggered configuration of the first and second rows of holes 60, 70 may be such that these rows of holes have hole pairs (*e.g.,* a first pair 60a, 70a and a second pair 60b, 70b) that define a respective axis pointing away from the central axis CA. However, in some embodiments, at least one hole pair may intersect central axis CA and/or intersect first plane P1 at a perpendicular angle.

The additional hole options provided by the second row 70 may also be utilized for connecting other components, such as fixation pins (not shown) to couple the rings 20, 30 to the respective bone fragments/segments. Still further, the staggered configuration of holes between the first and second rows 60, 70 may also help prevent interference between components attached to nearby holes. For example, a strut 40a-f attached to the first hole 70a of the second row 70 and a fixation pin, wire, or other fixation member attached to the first hole 60a of the first row 60 may not radially interfere with each other because of the staggering. It should also be understood that the size and/or number of tabs 50 may increase or decrease depending on the diameter of the rings 20 and 30, with greater diameter rings 20 and 30 having larger tabs 50 with more holes 70 compared to smaller diameter rings. For example, the illustrated tabs 50 include six holes 70, and a smaller ring may include smaller tabs 50 with four holes each, for example. Additionally, larger diameter rings 20, 30 may have more tabs 50 than smaller diameter rings 20, 30. For example, a larger diameter ring 20, 30 may have six tabs 50, while a smaller diameter ring 20, 30 may have three tabs 50.

As shown, each strut 40a-f may include a threaded portion 42 that may thread into or out of a tube portion 44, for example by interaction with a quick release mechanism 46, to decrease or increase the length, respectively, of the telescopic strut 40. In other embodiments, struts 40a-f may include an electric motor (not shown) for automated linear actuation to lengthen or shorten struts 40a-f. An example of such motorized struts can be found disclosed in U.S. Application No. 18/160,780, filed January 27, 2023, the disclosure of which is incorporated by reference herein in its entirety.

Each end of each strut 40a-f may be coupled to first ring 20 and second ring 30 via a joint mechanism 80 and secured via a nut 82. Joint mechanism may be a ball joint, a constrained hinge joint, or a universal joint, as illustrated. The use of universal joints on each end of the strut 40a-f provides for six degrees of freedom of motion of the external fixation system 10. It should be understood that although the disclosure is generally described in the context of closed circular rings, the concepts described herein may apply with equal force to other types of rings, such as open rings and/or U-shaped rings.

In external fixation system 10, telescopic struts 40a-f may be used to reduce fractures and correct deformities over time. Patients correct the deformities by incrementally adjusting struts 40a-f in accordance with a correction plan. In this regard, the lengths of the struts 40a-f may be adjusted over time to change the position and orientation of the two rings 20, 30 with respect to one another, which in turn repositions and reorients the bone fragments, with a goal of correcting the bone deformity. The adjustment of the external fixator 10 should strictly comply with the predetermined correction plan to ensure the desired clinical results are achieved.

Computational applications exist in which two-dimensional radiographic images of fixation frame 10 mounted on a bone may be utilized to determine the relative position of fixation frame 10 to bone fragments of the bone and to develop a correction plan for adjusting fixation frame 10 over time. An exemplary computational application for planning and optimizing bone correction is disclosed in U.S. Patent No. 8,654,150 ("the '150 Patent"), the disclosure of which is hereby incorporated by reference herein in its entirety. However, issues may arise at least due to a parallax effect between the 2D radiographic images and the 3D fixation frame. Thus, the scale and perspective of the 2D radiographic images may not be commensurate with the 3D fixation frame 10 resulting in inaccurate measurements. Thus, measurements obtained from these radiographic images may not accurately reflect the 3D positioning of rings 20 and 30 relative to each other and the underlying bone which may result in suboptimal correction plans and undesirable patient outcomes. These potential inaccuracies can be rectified by utilizing a reference device of known scale and 3D coordinates to calibrate the 2D radiographic images and to obtain accurate fixation frame parameters, such as ring offset measurements and relative pose and position of rings 20 and 30 relative to the bone. The following describes exemplary reference assemblies which may be connected to ring 20 and/or 30.

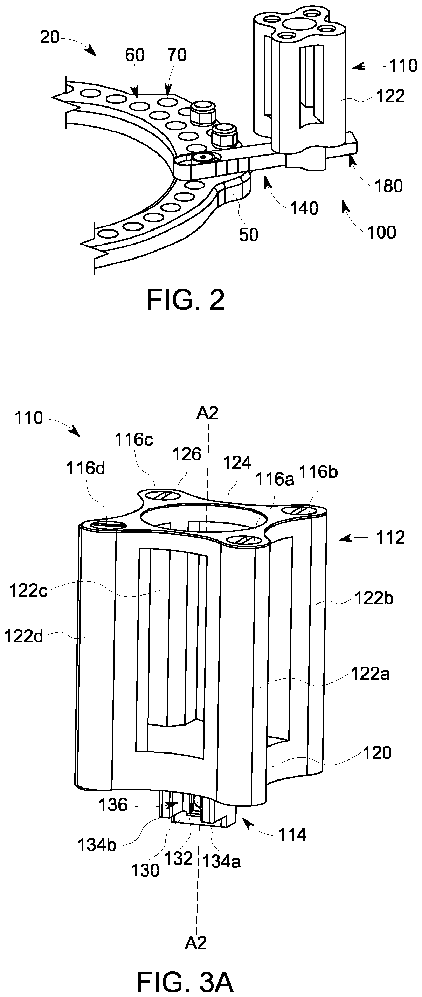

FIGS. 2-5D depict a reference assembly 100 according to an embodiment of the disclosure. Reference assembly 100 generally includes a reference device 110, a mount device 140, and a fastener 180.

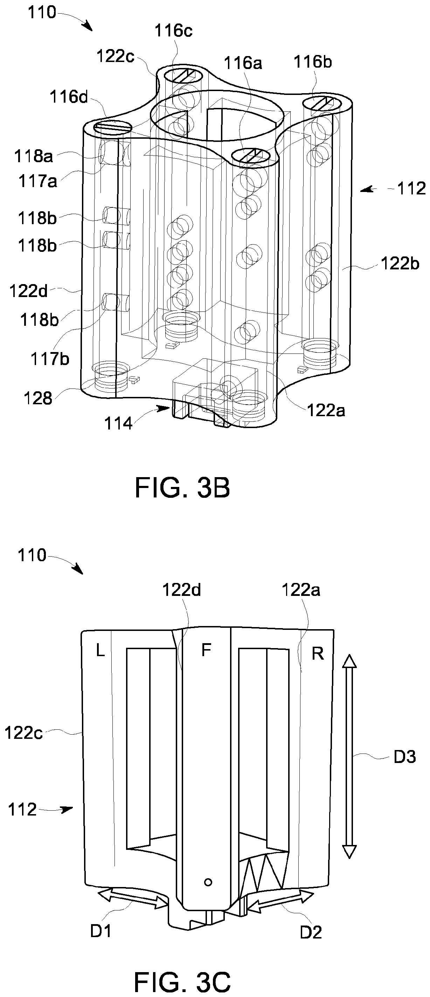

FIGS. 3A-3D depict reference device 110 which generally includes a reference body 112 and a connection member 114 extending from reference body 112. Reference body or reference array 112, as depicted, may include a base 120 and a plurality of reference columns 122 extending upwardly or superiorly from base. Each column 122 is interconnected at one end via base 114 and may also be interconnected via struts 124 at another end of columns 122 or somewhere else along their length, as shown in FIG. 3A. Regardless of where columns 122 are interconnected, such columns are generally in a fixed relationship relative to one another. In this regard, columns 122 may be arranged in a polygonal pattern about a longitudinal axis A1 of reference body 112. For example, in the embodiment depicted, reference body 122 may have four reference columns 122a-d arranged in a square-shaped pattern. As such, a first distance D1 (also referred to as a first width) measured between the first and second columns 122a, 122b and the third and fourth columns 122c, 122d may be equal to a distance D2 (also referred to as a second width) measured between the first and fourth columns 122a, 122d and the second and third columns 122a, 122c, as shown in FIG. 3C. Widths D1 and D2 may be 15 mm to 30 mm, for example. More specifically, widths D1 and D2 may be 17.67 mm or 28.28 mm, for example. In other embodiments, widths D1 and D2 may differ such that columns 122a-d are arranged in a rectangular pattern. In other embodiments, more or less reference columns 122 may be provided. For example, three reference columns 122 may extend from base 120 and form an equilateral triangle. In another example, five reference columns 122 may be arranged in a pentagon pattern. In either arrangement, columns 122 may form the corners of a polygonal pattern such that the reference columns 122 in the pattern are parallel to one another.

Also, as shown in FIG. 3C, each reference column 122a-d extends from base 120 a third distance D3 (also referred to as a column length) from base 120. In this regard, the column length D3 for each column 122a-d may be the same. For example, each column 122a-d may have a length of 30 mm to 60 mm. More specifically, the column length D3 for each column 122a-d may be 36 mm or 56 mm, for example. However, in some embodiments, the column length D3 may differ for each reference column 122a-d. In even further embodiments, the length of some of columns 122a-d may be equal, while the lengths of other such columns 122a-d may differ. For example, first and third columns 122a, 122c may have the same length while differing from the lengths of the second and fourth columns 122b, 122d, which themselves may have the same length. The differing lengths may help visually identify each reference column 122a-d.

Each reference column 122a-d may include a plurality of reference objects, such as a first reference object 118a and a second reference object 118b, for example. Reference objects 118a, 118b may be made from a radiopaque material such that it absorbs X-rays and appears opaque in a radiographic or other medical image. Examples of such radiopaque materials include radiopaque metals (*e.g.,* tantalum, tungsten, stainless steel, and zirconium), radiopaque ceramics (*e.g.,* zirconia and alumina), and/or radiopaque polymers (*e.g.*, polyoxymethylene or POM (aka Delrin® of Delrin USA LLC), barium sulfate and tantalum-loaded polymers). Reference objects 118a, 118b may alternatively be made from a radiolucent material such as a material that is entirely or partially transparent to radiation and/or mostly entirely or partially transparent in X-ray, fluoroscopy, and other imaging modalities. Examples of radiolucent materials can include polymers (*e.g.*, polytetrafluoroethylene or PTFE (aka Delrin® of Delrin USA LLC)), plastic, para-aramid synthetic fiber, resins (*e.g.*, polyether imide), carbon fiber or carbon composite, wood, cellulose, and the like. Polymers can include polypropylene, polyethylene, polyether ether ketone, polyaryletherketone, acrylonitrile butadiene styrene, nylon, and the like. Radiolucency of reference objects 118a, 118b can be adjusted by combining material having varying densities within the reference objects 118a, 118b. For example, a reference object 118a, 118b can include a first material with a higher density than a second material of reference object 118a, 118b. This first material may absorb more radiation and be less radiolucent than the second material, which may have a lower density and may absorb less radiation and be more radiolucent. Reference body 112 may also be made from a radiopaque or radiolucent material as desired. For example, reference objects may be made from stainless steel, while reference body 112 may be made from PTFE or POM.

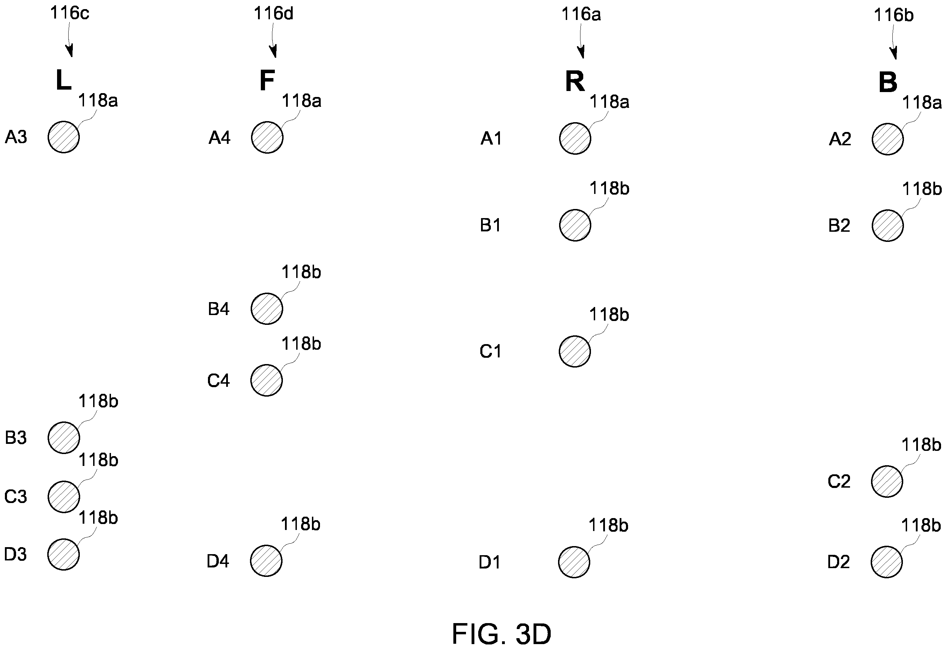

As shown in FIGS. 3B and 3D, each reference object 118a, 118b in each reference column 122a-d may be in the form of a sphere or bead. However, other shapes are contemplated, such as a cylinder, for example. Each spherical reference object 118a, 118b in each reference column 122a-d may have the same diameter. However, as best shown in FIG. 3B, first reference object 118a may have a larger diameter than second reference object 118b. For example, first reference object 118a may have a diameter of 4 mm, and second reference object 118b may have a diameter of 2 mm. Each reference column 122a-d may have a plurality of reference objects 118a and/or 118b. For example, each reference column 122a-d may have four of first reference object 118a, four of second reference object 118b, or four of any combination of first and second reference objects 118a, 118b. While four reference objects 118a, 118b are preferable as they may provide an optimal balance between accuracy and compactness of reference body 112, each reference column 122a-d may have more or less reference objects 118a, 118b (*e.g.,* three or five of first and/or second reference objects 118a, 118b). Additionally, it is preferable that one of the four reference objects be the larger diameter first reference object 118a, while the remaining three reference objects be the smaller diameter second reference object 118b, as illustrated in FIGS. 3B and 3D. Although, other configurations are contemplated, such as two of first reference object 118a and two of second reference object 118b, for example. The size difference between the larger spherical object 118a and smaller spherical objects 118b may help determine an appropriate scale of a radiographic image containing the reference objects 118a, 118b. In other words, when a two-dimensional radiographic image of reference columns 122a-d is obtained, the scale of the image may not be known. However, knowing the diameters of first and second reference objects 118a, 118b and their relative size differences can help calibrate the scale of the radiographic image ensuring proper measurement of the other objects within the radiographic image (*e.g.,* bone and fixation frame 10).

Reference objects 118a, 118b are preferably colinearly arranged within their respective column 122a-d and at various longitudinal positions. In other words, first refence column 122a may have reference object positions A1-D1 distributed along longitudinal axis of column 122a, second reference column 122b may have reference object positions A2-D2 distributed along a longitudinal axis of second column 122b, third reference column 122c may have reference object positions A3-D3 distributed along a longitudinal axis of third column 122c, and fourth reference column 122d may have reference object positions A4-D4 along a longitudinal axis of fourth column 122d. It should be noted that the distances between each object position mentioned above is measured center-to-center of the object spheres 118a, 118b that occupy such positions. As shown in FIG. 3D, the larger diameter first reference object 118a may occupy each of the first positions A1-A4, and second reference objects 118b may respectively occupy the remaining positions B1-B4, C1-C4, and D1-D4. Positions A1-A4 may be located furthest from base 120, positions D1-D4 may be located closest to base 120, and positions B1-B4 and C1-C4 may be located somewhere in-between. It is preferable that the larger diameter first reference object 118a be located at the first position A1-A4 as it may help identify and distinguish each reference column 122a-d in a radiographic image from the others. It is, however, contemplated that first reference object 118a may be located at the fourth position D1-D4, for example.

The reference objects 118a that occupy first positions A1-A4 may be arranged in the same plane which may be perpendicular to axis A2. In other words, first positions A1-A4 may be coplanar. Similarly, fourth positions D1-D4 may be coplanar. Thus, the respective distances between first positions A1-A4 and fourth positions D1-D4 may be the same. However, in some embodiments, these positions may be staggered between the columns 122a-d. Although first positions A1-A4 and fourth positions D1-D4 may be co-planar and equidistant, second positions B1-B4 and third positions C1-C4 may not. In other words, the distances between second positions B1-B4 and first positions A1-A4 may differ from reference column 122a-d to reference column 122a-d such that second positions B1-B4 are longitudinally staggered from column to column. Similarly, the distances between third positions C1-C4 and first positions A1-A4 may differ from reference column 122a-d to reference column 122a-d such that third positions C1-C4 are longitudinally staggered from column to column. However, in some embodiments, some of second positions B1-B4 or third positions C1-C4 may be coplanar (*see e.g.,* FIG. 3D reference columns 116a and 116b). In such embodiments third positions C1-C4 or second positions B1-B4 should then be staggered so that ultimately each column has a different longitudinal reference object pattern. This is depicted in FIG. 3D in which, even though B1 and B2 are coplanar, C1 and C2 are staggered.

The relative and differing distances of reference object positions A1-A4, B1-B4, C1-C4, and D1-D4 for reference columns 122a-d may help identify and distinguish each reference column 122a-d from the others in a radiographic image which can then help identify the orientation of the image. This may be done mathematically. For example, each column 122a-d may have a cross-ratio distinct from the others. The cross-ratio may be defined as follows:

$$CrossRatio = \frac{AC * BD}{BC * AD}$$

AC is the distance between respective positions A1-A4 and C1-C4, BD is the distance between respective positions B1-B4 and D1-D4, BC is the distance between respective positions B1-B4 and C1-C4, and AD is the distance between respective positions A1-A4 and D1-D4. Thus, as exemplified in the tabulated example below in table 1, the cross-ratio for each column 122a-d may differ which may allow a processor to automatically measure the distances between reference objects 118a, 118b, using pixel units, for example, and calculate the cross-ratios which may then be cross-referenced with the known cross-ratios of each column 122a-d for automatic identification. Locating the larger diameter first reference object 122a at the first positions A1-A4 may help the processor identify the first position A1-A4 so that the other positions can be measured therefrom.

| TABLE 1 | | | |
| --- | --- | --- | --- |
| 1st Column (116a) | 2nd Column (116b) | 3rd Column (116c) | 4th Column (116d) |
| A1B1 = 0.5 units | A2B2 = 0.5 units | A3B3 = 2.25 units | A4B4 = 1.25 units |
| B1C1 = 1 units | B2C2 = 2 units | B3C3 = 0.375 units | B4C4 = 0.5 units |
| C1D1 = 1.5 units | C2D2 = 1.5 units | C3D3 = 0.375 units | C4D4 = 1.25 units |
| A1C1 = 1.5 units | A2C2 = 2.5 units | A3C3 = 2.625 units | A4C4 = 1.75 units |
| B1D1 = 2.5 units | B2D2 = 2.5 units | B3D3 = 0.75 units | B4D4 = 1.75 units |
| A1D1 = 3 units | A2D2 = 3 units | A3D3 = 3 units | A4D4 = 3 units |
| Cross-Ratio = 1.25 | Cross-Ratio = 1.04 | Cross-Ratio = 1.75 | Cross-Ratio = 2.04 |

Reference objects 118a, 118b may be embedded in each reference column 122a-d in the desired longitudinal pattern. For example, a radiolucent polymer material forming reference body 112 may be injected molded about each reference object 118a, 118b in the desired longitudinal arrangement, as described above. However, as shown in FIG. 3B, each reference column 122a-d may be comprised of a longitudinal opening 126 and a reference insert removably 116a-d disposed within the respective openings 126. In this regard, reference body 112 may be made from a radiolucent metal or polymer and machined or otherwise manufactured to have openings 126. Each reference insert 116a-d may include a plurality of transverse openings 117a, 117b configured to receive the reference objects 118a, 118b at the desired longitudinal intervals corresponding to the reference object positions A1-A4, B1-B4, C1-C4, and D1-D4, described above. For example, as shown, a fourth reference insert 116d may be in the form of a cylinder with a plurality of cylindrical transverse openings 117a, 117b extending therein at predetermined positions along a length thereof. Spherical reference objects 118a, 118b may be inserted into the respective transverse openings 117a, 117b and a curable material, such as an adhesive, or a set screw, for example, may be used to secure the reference objects 118a, 118b within their respective transverse openings 117a, 117b. Such transverse openings 117a, 117b may be configured for the specific reference object 118a, 118b placed therein. Thus, for example, a larger diameter transverse opening 117a corresponding to the larger diameter spherical reference object 118a may be formed at one end of the insert 116d while smaller diameter transverse openings 117b may be formed at other positions along the length of insert 116d for smaller diameter spherical reference objects 118b. Alternatively, each insert 116a-d itself may be injected molded with reference objects 118a, 118b embedded therein.

A first end of each reference insert 116a-d may include a tool feature configured to engage an insertion tool, such as a driver, and a second end of each insert may be a threaded end 128 for threaded engagement with a corresponding column opening 126 to secure reference inserts 116a-d to reference body 112. Thus, each insert 116a-d may be threadedly connected to reference body 112. However, other connection mechanisms are also contemplated, such as press-fit and snap-fit connection mechanisms, for example. The removable insert configuration depicted allows reference body 112 to be configured with a variety of different reference object patterns, as desirable.

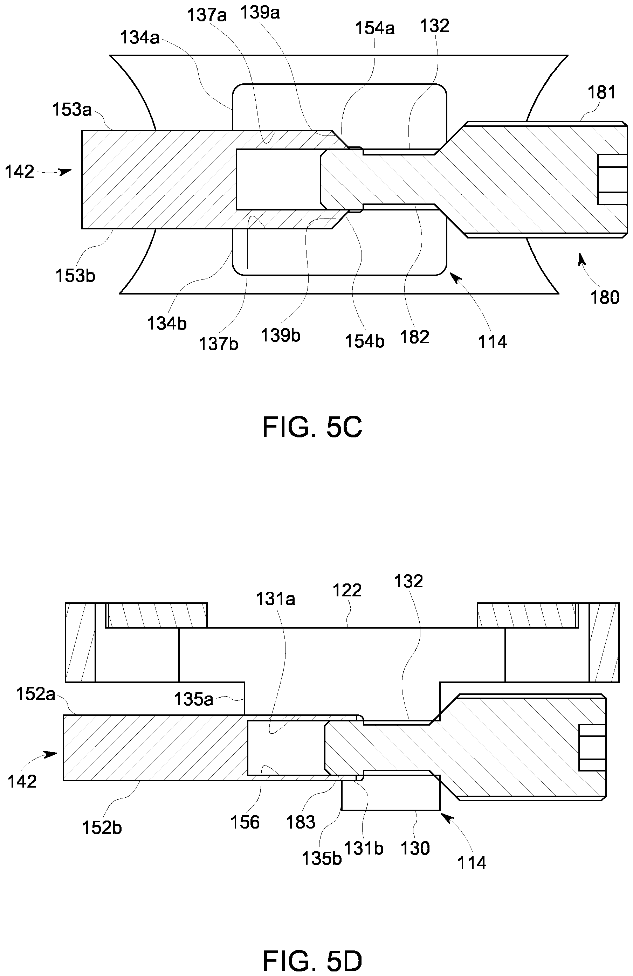

Connection member 114 may extend from base 120 in a direction opposite reference columns 122a-d and may be configured to connect to mount device 140. In this regard, connection member 114 may define a recess 136 configured to receive at least a portion of mount device 140 and a through-hole 132 in communication with recess 136 for receipt of at least a portion of connector 180, as best shown in FIGS. 3A, 5C, and 5D. As shown, recess 136 may be defined by a plurality of intersecting walls which engage and help constrain mount device 140 when disposed within recess 136. In this regard, connection member 114 may include an upper wall 135a, a lower wall 135b, an end wall 130, and first and second sidewalls 134a, 134b, as best shown in FIGS. 5C and 5D. Upper wall 135a may be comprised by base 120, and lower wall 135b may be comprised by at least a portion of end wall 130. Sidewalls 134a, 134b define parallel side surfaces 137a, 137b, and upper and lower walls 135a, 135b define opposing upper and lower surfaces 131a, 131b, as also shown in FIGS. 5C and 5D. Further, in the embodiment depicted, first and second chamfer surfaces 139a, 139b (also referred to as angled surfaces) may be defined by end wall 130 and intersect first and second side surfaces 137a, 137b of sidewalls 134a, 134b, as best shown in FIG. 5C. Through-hole 132 extends through end wall 130 between chamfered surfaces 139a, 139b , as also shown in FIG. 5C.

Recess 136 may also perform an indexing function to help arrange reference columns 122a-d in a desired orientation relative to mount device 140 so that, when reference device 110 is mounted to a ring 20, 30 of fixation frame 10, reference body 112 is positioned in an optimal orientation for radiographic imaging, as described further below. In this regard, first and second sidewalls 134a, 134b may be parallel to the first width D1 of reference body 112 extending between first and second columns 122a, 122b and third and fourth columns 122c, 122d. Additionally, upper and lower walls 135a, 135b may extend perpendicular to column length D3 and longitudinal axis A2.

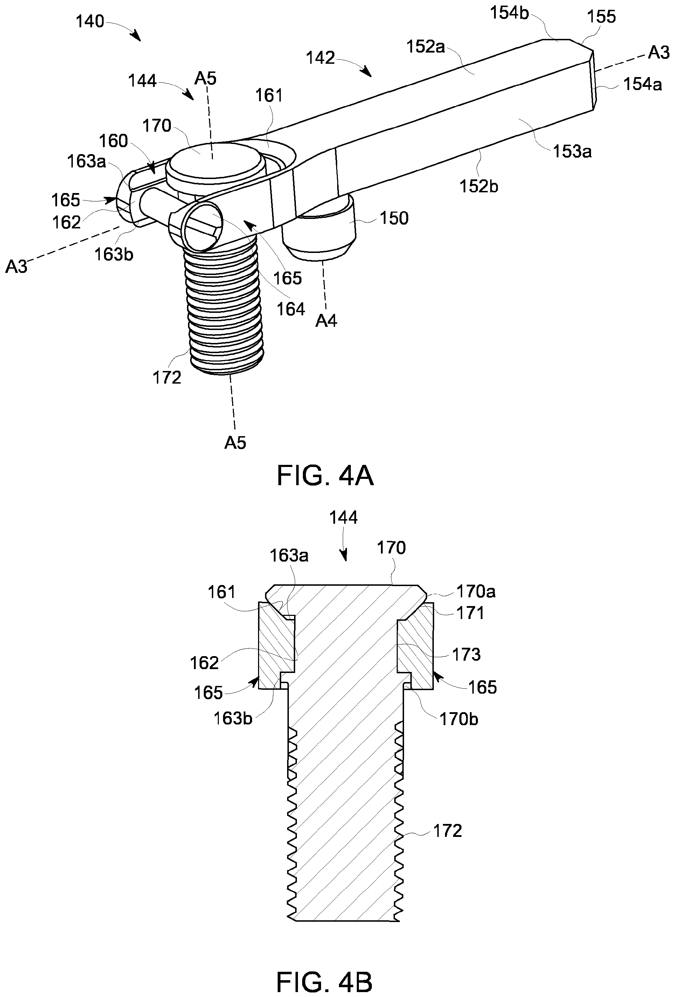

FIGS. 4A and 4B depict a mount device 140 according to an embodiment of the disclosure. Mount device or connection device 140 generally includes a mount body 142 and a dynamic member 144 moveable relative to mount body 140.

Mount body or baseplate 142 may be an elongate structure extending along a longitudinal axis A3 from a first end to a second end thereof. The first end may include a planar end surface 155 that may be oriented perpendicular to longitudinal axis A3 and a threaded hole 156 (see FIG. 5D) extending through end surface 155. The first end may also have chamfer surfaces 154a, 154b (also referred to as angled surfaces) that may intersect end surface 155 at an oblique angle. Such chamfer surfaces 154a, 154b may be optional. Mount body 142 may also have a square or rectangular cross-section such that it has planar and parallel upper and lower surfaces 152a, 152b and planar and parallel side surfaces 153a, 153b. Side surfaces 153a, 153b may intersect first and second chamfer surfaces 154a, 154b, respectively, while upper and lower surfaces 152a, 152b may intersect end surface 155 and first and second chamfer surfaces 154a, 154b, as best shown in FIG. 4A. While the square or rectangular cross-sectional geometry of mount body 142 is preferable to help orient and constrain reference device 110 when connected thereto, other geometries may be provided. For example, upper and lower surfaces 152a, 152b and side surfaces 153a, 153b may be convex or concave and/or may be tapered such that they are not parallel. Additionally, the first end of mount body 142 may not have chamfer surfaces 154a, 154b, but may instead have one or more indentation or projection (not shown) that may key into or with a corresponding feature in reference device 110 when connected thereto to help constrain such engagement.

A locating pin or static member 150 may extend downward or inferior from lower surface 152b at a location between the first and second end of mount body 142. Locating pin 150 may define an axis A4 which may be oriented perpendicular to longitudinal axis A3. Locating pin 150 may be immoveable or static relative to mount body 142 and may be configured to be received within a hole 60a, 60b, 70a, 70b in ring 20 or 30 of fixation frame 10. In some embodiments, locating pin 150 may include a stabilizing feature (not shown) to help stabilize locating pin 150 within the respective hole 60a, 60b, 70a, 70b to help limit movement of locating pin 150 therein. Such stabilizing feature can include an O-ring or flexible legs for a snap-fit connection, for example.

The second end of mount body 142 may include a pair of arms 165 and a slot 160 defined between the arms 165. Arms 165 (also referred to as prongs) may extend in the longitudinal direction and may be arranged parallel to each other and to longitudinal axis A3. Each arm 165 may include an inwardly extending rail 162 that may define an upper shoulder or abutment surface 163a and a lower shoulder or abutment surface 163b. Mount body 142 may also include a chamfer surface 161 extending along each of arms 165 at an upper side thereof, as best shown in FIG. 4A. Chamfer surface 161 may intersect upper shoulder 163a, as shown in FIG. 4B, and may also extend in a U-shaped path about slot 160, as shown in FIG. 4A. Slot or recess 160 may extend in a longitudinal direction along longitudinal axis A3 and may also extend through upper and lower surfaces 152a, 152b of mount body 142. A fastener 164 may be connected to arms 165 and extend across slot 160 which may help retain dynamic member 144 within slot 160, as discussed further below. However, in some embodiments, mount body 142 may encircle or enclose slot 160. Arms 165 may be flexible such that fastener 164 can be used to flex arms 165 toward each other to narrow slot 160, and to flex arms 165 away from each other to widen slot 160.

Dynamic member or dynamic pin 144 may include a head 170 and a shaft 172 extending from head 170 along a longitudinal axis A5. As shown in FIG. 4B, head 170 may include an upper head portion 170a, a lower head portion 170b, and a circumferential groove extending about axis A5 disposed between upper and lower head portions 170a, 170b. Upper head portion 170a may be constrained from falling through slot 160 by upper abutment surface 163a and may include a circumferential chamfer surface 171 correspondingly angled to slidingly engage chamfer surface 161 of mount body 142. The corresponding chamfers 161 and 171 of mount body 142 and dynamic member 144 may help auto-align dynamic pin 144 within a hole in ring 20, 30. Lower head 170b portion may form a circumferential collar extending radially outwardly and may constrain head 170 from being lifted out of slot 160 in conjunction with lower abutment surface 163b. Circumferential groove 173 may be defined between upper and lower head portions 170a 170b. Such groove 173 may be configured to receive rails 162 of first and second arms 165. Thus, when head 170 is received within slot 160, axis A5 of dynamic member may be parallel to axis A4 of locating pin 150, and head 170 may be slideable in the longitudinal direction along axis A3 closer to or further away from axis A4 of locating pin 150. This allows mount device 140 to adapt to rings 20, 30 of different sizes and/or ring holes 60a, 60b, 70a, 70b separated by different distances. Shaft 172 may be a threaded shaft configured to be slidingly received within a hole 60a, 60b, 70a, 70b in first or second ring 20, 30. A nut, such as nut 82 shown in FIG. 1, may be threadedly engaged to threaded shaft 172 when disposed within a hole 60a, 60b, 70a, 70b in first or second ring 20, 30 to secure dynamic member 144 member to ring 20, 30.

Fastener or connector 180 generally includes a head 181 and a shaft 182. Head 181 may be a thumb knob that allows for manual operation. However, in some embodiments, head 181 may have a tool feature for engagement with a driver or wrench, for example. Shaft 182 may include a threaded end 183 (see FIG. 5D) such that a portion of shaft 182 is unthreaded and another portion is threaded. However, shaft 182 may be threaded along a majority or entirety of its length.

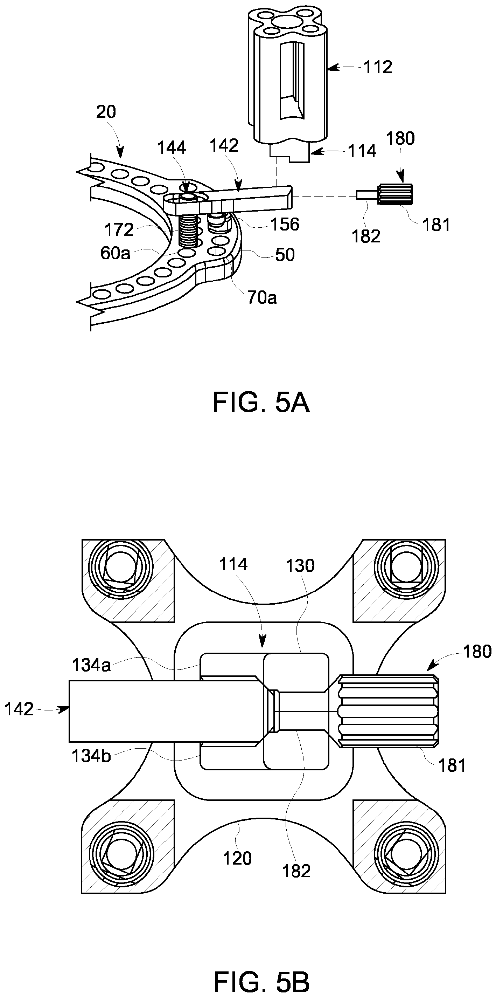

FIGS. 5A-5D depict the connection of reference assembly 100 to first ring 20 of fixation frame 10. It should be understood though that such connection may also be applicable to second ring 20. As shown, mount device 140 may be mounted or connected to first ring 20 by inserting locating pin 150 and dynamic member 144 into respective holes of the staggered rows 60, 70 of ring 20. More specifically, in the embodiment depicted, locating pin 150 may be inserted into a first hole 70a of second row of holes 70, and dynamic member 144 may be inserted into a first hole 60a in first row of holes 60, as shown in FIG. 5A. As the dynamic member 144 and locating pin 156 are inserted into their respective holes 60a, 70a, dynamic member 144 may be moved longitudinally within slot 160 as necessary to ensure that shaft 172 of dynamic member 144 is properly aligned with first hole 60a. In this regard, reference assembly 100 is adapted to hole pairs of differing distances. It should be noted that, in this configuration of mount device 140 with respect to ring 20, when reference device 110 is connected to mount device 140, reference body 112 is positioned outside of the ring diameter of ring 20 rather than inside the ring diameter, as illustrated in FIGS. 2 and 5A. This is preferable as the patient's limb is located within ring 20 and may obstruct reference assembly 100. However, it is contemplated that locating pin 150 may be inserted into a hole in the inner ring of holes 60 (*e.g.,* first or second hole 60, 60b) and the dynamic member 144 may be inserted into a hole in the outer ring of holes 70 (*e.g.,* first or second hole 70a, 70b) so that reference device 110 ends up positioned within the diameter of ring 20 when connected to mount device 140. Nonetheless, once dynamic pin 144 is positioned within its corresponding hole, a nut, like nut 82 of FIG. 1, may be threaded onto threaded shaft 172 to secure dynamic member 144 and mount body 142 to ring 20. Chamfer surfaces 161, 171 of mount body 142 and dynamic member head 170 may engage each other as the nut is tightened which may help auto-align threaded shaft 172 with the hole so that shaft 172 is coaxial with hole and so that axes A4 and A5 are parallel.

After mount device 140 is mounted to ring 20 or prior to mounting mount device 140 to ring 20, reference device 110 is connected to the first end of mount body 142. In this regard, the first end of mount body 142 may be inserted into recess 136 of connection member 114 of reference device 112 so that reference columns 122a-d extend upwardly therefrom. However, it is contemplated that reference device 110 may be connected to mount body 142 so that reference columns 122a-d extend downwardly. As the first end of mount body 142 is inserted into recess 136 of connection member 114, upper surface 152a of mount body 142 engages upper surface 131a of connection member 114, lower surface 152b of mount body 142 engages lower surface 131b of connection member 114, side surfaces 153a, 153b of mount body 142 engage side surfaces 137a, 137b of connection member 114, chamfer surfaces 154a, 154b of mount body 142 engage chamfer surfaces 139a, 139b of connection member 114, and threaded opening 156 of mount body 142 is aligned with through-hole 132 of connection member 114, as best shown in FIGS. 5B-5D. Thereafter, threaded end 183 of connector 180 is inserted through through-hole 132 and into threaded engagement with threaded hole 156 of mount body 142 which draws mount body 142 into further engagement with chamfer surfaces 139a, 139b of connection member 114.

This collection of surface engagement helps constrain reference device 110 from all rotational degrees of freedom relative to mount body 142 and indexes reference body 112 into a desired orientation relative to ring 20. As mentioned above, first and second rows of holes 60, 70 may have a staggered arrangement. Thus, when mount device 140 is connected to hole pairs of first and second rows 60, 70, the longitudinal axis A3 of mount body 142 may extend at the angle Θ relative to plane P1. This may have the effect of rotating or orienting reference body 112 so that in a M-L plane and/or in an A-P plane, at least three reference columns are presented (one column may be obscured by another). This is preferable over an orientation in which only two columns 122 of columns 122a-d are presented in the A-P and/or M-L planes (two columns 122 may be obscured by the other columns 122) which could occur if mount body 142 were to be positioned on ring 20 so that axis A3 intersects central axis CA perpendicular to plane P1. As such, it is preferable for reference body 112 to be oriented relative to ring so that A-P and M-L radiographic images thereof include at least three visible reference columns 122 of the four reference columns 122a-d. Thus, while the depicted embodiment is shown connected to first holes 60a, 70a of first and second rows of holes 60, 70, it should be understood that other hole pairs (*e.g.,* holes 60b and 70b) may be selected depending on availability (other hole pairs may be occupied with other devices) provided that at least three reference columns 122 are apparent in the desired radiographic images. Additionally, the connection between mount body 142 and reference device 110 is such that longitudinal axis A2 of reference body 112 is parallel with central axis CA of ring.

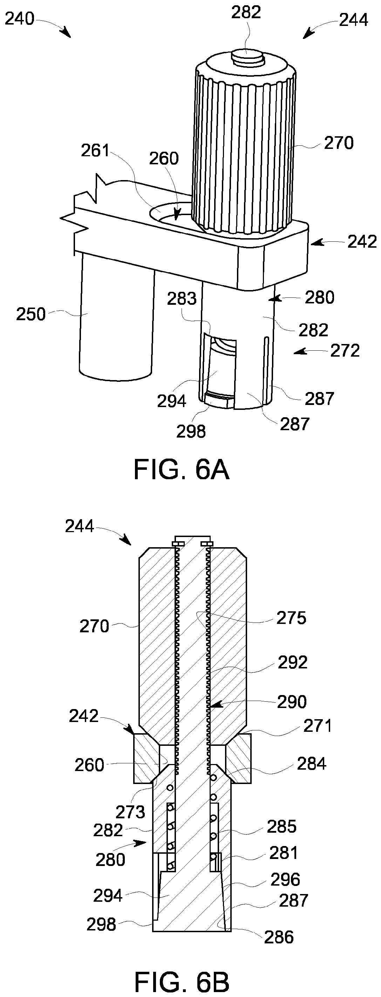

FIGS. 6A and 6B depict a mount device 240 according to another embodiment of the disclosure. Mount device 240 is similar to mount device 140. Thus, for ease of review, like elements are accorded like reference numerals to that of mount device 140 but within the 200-series of numbers. For instance, mount device 240 includes a mount body 242, locating pin 250, and a dynamic member 244. However, mount body 242 and dynamic member 244 may differ from mount body 142 and dynamic member 144, as described below.

Dynamic member 244 generally includes a head 270 and an expandable shaft 272 extending from head 270. Head 270 may be a thumb knob such that it may be operated manually. Alternatively, or in addition to, head 270 may include a tool feature for engagement with a corresponding tool, such as a driver or a wrench. For example, an exterior of head 270 may include a plurality of angled surfaces (not shown) for engagement with a wrench. Head 270 may include a threaded hole 275 extending entirely therethrough or at least partially therein. A lower end or inferior end of head 270 may include a circumferential chamfer surface 271 configured to engage superior chamfer surface 261 of mount body 242 to help auto-align dynamic member 244 to a hole in ring 20 or 30, as described above with respect to mount device 140.

Expandable shaft 272 may include an outer member 280 and an inner member 290. Inner member 290 may be moveable relative to outer member 280 such that movement of inner member 290 radially expands or radially contracts outer member 280. As shown, outer member or sleeve 280 may include a sleeve body 282 and a plurality of legs 287 extending downwardly or inferiorly relative to sleeve body 282. Outer member 280 may include two legs 287, for example. However, in some embodiments more than two legs 287 may be provided, such as three legs 287, for example. Legs 287 may be cantilevered to sleeve body 282 and may be flexible such that they are radially moveable from a relaxed position, as shown in FIG. 6B, to a flexed position in which legs 287 are positioned radially outwardly from the relaxed position. Thus, legs 287 may be biased toward the relaxed position. Legs 287 may each include an inner surface 286 that may taper or be angled radially outwardly from a superior to inferior direction, as best shown in FIG. 6B. Thus, such inner surfaces 286 of legs 287 may at least partially define a cavity 281 that has a larger cross-sectional dimension at an inferior end of legs 287 *(i.e.,* free end of legs 287) than at a superior end of legs 287 *(i.e.,* end of legs 287 connected to sleeve body 282). Sleeve body 282 may include a circumferential chamfer surface 284 at an upper end or superior end thereof. Such chamfer surface 284 may be configured to engage an inferior chamfer surface 273 of mount body 242, as shown in FIG. 6B. Such corresponding chamfer surfaces 273, 284 may further assist in auto-alignment of dynamic member 244.

Inner member or plunger 290 may include a plunger body 294 and a threaded shaft 292 extending from plunger body 294. Plunger shaft 292 may be a threaded shaft that may extend through slot 260 of mount body 242 and threadedly engage threaded hole 275 of head 270. Plunger body 294 may be disposed within cavity 281 of outer member 280 and may include a tapered outer surface 296 that may be correspondingly tapered with respect to inner surfaces 286 of legs 287 such that outer surface 296 of plunger body 294 tapers radially outwardly from a superior to inferior direction. A foot or guide projection 298 may extend radially outwardly from plunger body 294 and may be slidably disposed within a window or slot 283 of outer member 280 so as to help translationally guide plunger 290 and prevent its rotation relative to outer member 280.

In operation, dynamic member 244 may slide within slot 260 to help align itself to a hole in ring 20, 30, as described above with respect to dynamic member 144. Once dynamic member 244 is positioned within its respective ring hole, head 270 may be rotated in a first direction which may cause plunger body 294 to move upwardly within cavity 281 which may concurrently cause outer surface 296 of plunger body 294 to push on inner surfaces 286 of legs 287 thereby pushing legs 287 radially outwardly into engagement with ring 20, 30. Dynamic member 244 can then be removed by rotating head 270 in a second direction which may drive plunger body 294 downwardly allowing legs 297 to move radially inwardly to their relaxed state. In other words, expandable shaft 272 may have a first configuration and second configuration. In the first configuration, plunger body 294 may be positioned at an inferior end of cavity 281 such that legs 287 may be in their relaxed state. In the second configuration, plunger 294 may be positioned near a superior end of cavity 281 such that plunger body 294 may push against legs 287 radially outwardly to their flexed and expanded state. In the first configuration, expandable shaft 272 may be easily slid into and out of a hole of ring 20 or 30. In the second configuration, expandable shaft 272 via legs 287 may bear against ring 20, 30 to secure and stabilize expandable shaft 272 thereto. Although the embodiment depicted illustrates an example in which plunger 290 moves superiorly to expand legs 287 radially outwardly, it is contemplated that inner surfaces 286 of legs 287 and outer surface 296 of plunger body 294 may be oppositely configured such that driving plunger body 294 inferiorly or downwardly expands legs 287. A compression spring 285 may be positioned between outer member 280 and plunger body 294 to help stabilize plunger body 294 as it is moved from the first configuration to the second configuration and to help provide tactile feedback and to assist moving plunger body 294 back to the first configuration.

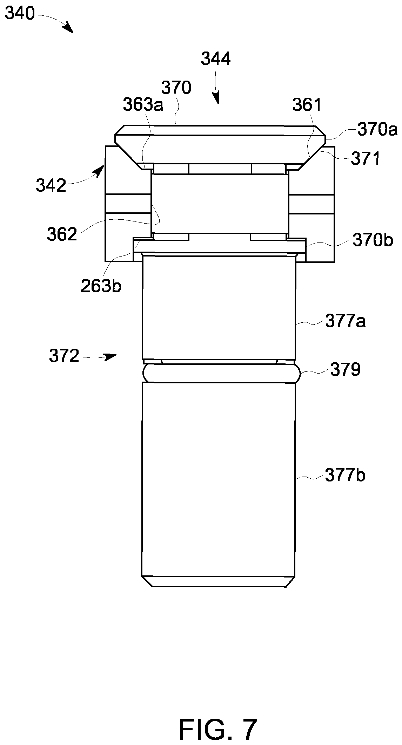

FIG. 7 depicts a mount device 340 according to a further embodiment of the disclosure. Mount device 340 is similar to mount device 140. Thus, for ease of review, like elements are accorded like reference numbers to that of mount device 140 but within the 300-series of numbers. For instance, mount device 340 includes a mount body 342 and a dynamic member 344. However, dynamic member 344 may differ from dynamic member 144, as described below.

Dynamic member 344 generally includes a head 370 and a shaft 372. Head 370 is similar to head 170 in that it includes a first head portion 370a and second head portion 370b configured to slide along opposing rails 362 of mount body 342. Additionally, mount body 342 is similar to mount body 142 in that it includes a chamfer surface 371 and rails 362 that define upper and lower abutment surfaces 363a, 363b. However, shaft 372 differs from shaft 172 in that it may include a first shaft portion or upper portion 377a, a second shaft portion or lower portion 377b, and an O-ring 379 positioned within a circumferential groove between first portion 377a and second portion 377b. In the embodiment depicted, first portion 377a and second portion 377b are fixed relative to each other, and O-ring 379 has a diameter slightly greater than that of first and second shaft portions 377a, 377b. Thus, in operation, when shaft 372 is pushed into a hole in ring 20 or 30 of fixation frame 10, O-ring 379 engages such hole and helps stabilize shaft 372 from movement therein via friction between O-ring 379 and ring 20, 30.

However, in other embodiments, second portion 377b may be moveable relative to first portion 377a, such as via a threaded member (not shown) extending through head 370 and first portion 377a to second portion 377b. In this regard, operating the threaded member may cause the second portion 377b to move toward first portion 377a to compress and outwardly expand O-ring 379. Thus, in such embodiment, when shaft 372 is initially positioned within a hole in first or second ring 20, 30, shaft 372 may have some play within the hole which can be reduced or eliminated by moving second portion 377b relative to first portion 377a to selectively expand O-ring 379.

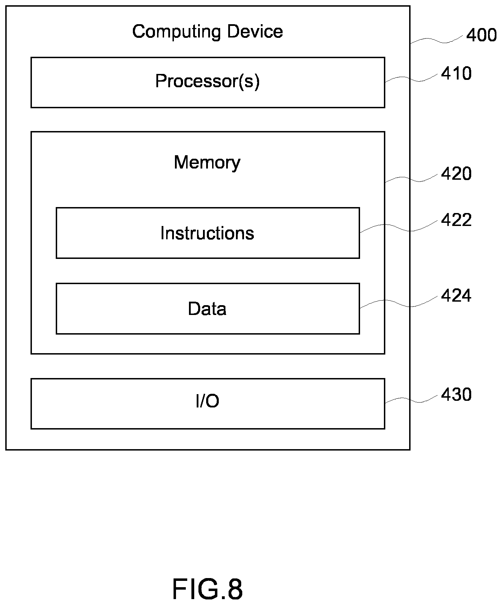

FIG. 8 depicts an exemplary computing device 400 that may be utilized to implement aspects of the present disclosure. Computing device 400 may include one or more processors 410, memory 420, one or more input/output "I/O" devices 430, and other components typically present in general purpose computing devices. The processor(s) 410 can be any conventional processor, such as a commercially available CPU. Alternatively, the processor 410 can be a dedicated component such as an application specific integrated circuit ("ASIC") or other hardware-based processor. Memory 420 can store information accessible by processor 410, including instructions 422 that can be executed by processor 410. Memory 420 can also include data 424 that can be retrieved, manipulated, or stored by processor 410. Memory 420 can be of any non-transitory type capable of storing information accessible by processor 410, such as a hard-drive, memory card, ROM, RAM, writecapable, and read-only memories. Instructions 422 can be any set of instructions to be executed directly, such as machine code, or indirectly, such as scripts, by processor 410. In that regard, the terms "instructions," "application," "steps," and "programs" can be used interchangeably herein. The one or more I/O devices 430 can include user input devices (*e.g.,* a mouse, keyboard, or touch screen) and one or more displays (*e.g.*, monitor having a screen, a touch-screen, a projector, or other device that is operable to display information).

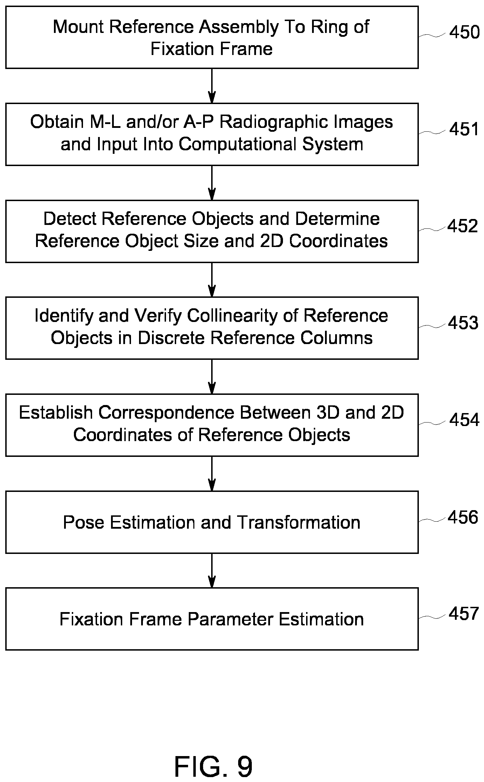

FIG. 9 depicts a method of obtaining fixation frame parameters for assessing position relative to bone and for generating a correction plan. The methods described herein may be implemented at least partially by a computational application of computing device 400. Such computational application may be a modified version of the aforementioned computational application of the '150 Patent, for example, to perform the methods described herein.

At block 450, reference assembly 100 may be connected to ring 20 of fixation frame 10, as described in more detail above. In some embodiments, reference assembly 100 could also be connected to ring 30. In even further embodiments, a first reference assembly 100 may be connected to first ring 20, and a second reference assembly connected to ring 30.

At block 451, an M-L and/or A-P radiographic image of fixation frame 10, reference assembly 100, and the underlying bone may be obtained and uploaded to the computational application. As described in more detail above, the orientation of reference body 112 may be such that at least three reference columns 122a-d are presented in one or both of these planes.

At block 452, processor 410 may detect reference objects 118a, 118b within the radiographic image and may determine their dimensions and 2D coordinates within the radiographic image with a distinction between the larger first reference objects 118a and smaller second reference objects 118b. Such dimensions and coordinates may be measured in pixel units. The detection of reference objects 118a, 118b in the radiographic image may include using an edge detection algorithm, such as Canny edge detection, for example. Additional image processing may be performed thereafter. For example, a Ramer-Douglas-Peucker algorithm may be utilized to help contour the items captured in the radiographic image. Once the requisite image pre-processing has been performed and the boundaries of the reference objects 118a, 118b identified, radius dimensions of each detected reference object and the 2D coordinates thereof may be determined by processor 410.

Blocks 453 and 454 represent a correspondence algorithm in which each detectable reference column 122a-d is first identified and verified for further use in the method. In this regard, processor 410 may find a first reference object, preferably the large reference object 118a, in a column 122 and fit a line to identify each set of colinear reference objects 118a, 118b. The coordinates of the larger first reference objects 118a, as determined in block 452, may assist in helping to define the first point in the colinear columns 122a-d via which the remaining reference objects 118b may be identified. Once an initial estimate of the reference objects 118a, 118b included in each detectable reference column 122a-d is made by processor 410, the collinearity of reference objects 118a, 118b may be assessed using the cross-product of the reference object positions A1-A4, B1-B4, C1-C4, D1-D4 to verify that the reference objects 118a, 118b estimated for each reference column 122a-d are indeed a part of that reference column. If the cross-product is less than a threshold value, such as 0.05, for example, then further verification is performed. If the threshold value is exceeded, then the reference objects identified do not have the requisite co-linearity. As such, processor 410 may select other reference objects 118a, 118b for co-linear assessment or another radiographic image may be utilized. Should the cross-product be less than the threshold value, then the cross-ratios of the respective co-linear columns 122a-d are computed, as described in more detail above. If the computed cross-ratios are close to the actual cross-ratios for reference objects 118a, 118b of each respective reference column 122a-d, then the reference columns 122a-d in the radiographic image have been verified for collinearity, distinctness, and for accuracy so that they may then be used to establish correspondence between the 2D coordinates of the image and the 3D coordinates of reference device 110 and the other items in the radiographic image, such as fixation frame 10.

As mentioned above, the first reference object 118a in each column 122a-d may be larger in cross-sectional dimension than the other reference objects 118b. This helps ensure the cross-ratio for any given column 122a-d is computed in the appropriate order for further analysis. For example, consider four reference objects 118 in a sequence A, B, C, and D which has a known cross-ratio. If processor 410 were to identify the reference objects A-D out of order (*e.g.,* in the order of D, C, B, and A) and compute the cross-ratio accordingly, then the out-of-order cross-ratio would not match that of the reference objects 118 in the proper sequence. Locating the larger reference object 118a at a known location within a reference column 122a-d, preferably at one of the end positions, helps reduce error and provides a mode of self-verification. This also helps reduce computation and matching across the potential different ratio combinations to find the right match on a particular column 122. Moreover, the relative sizes of the reference objects 118a and 118b may be used to scale the radiographic image upon initial detection by processor 410 so as to help in computing the distances in the image. This may be done by computing the pixel to mm (or other unit of measurement) ratio.

Thus, processor 410 may find the first and largest reference objects 118a in the radiographic image, scale the image, and fit a line to each reference object 118a to identify respective columns 122a-d. Using the reference objects 118a, 118b on the line, the cross-ratio for each column 112a-dd may be computed in a sequence starting from the largest reference object 118a. The cross-ratios of the columns 122a-d in the radiographic image may then be matched to the known cross-ratios of the reference columns 122a-d of reference device 110 to uniquely identify each reference column 122a-d. This may assist with further computations, such as a pose computation, as correspondence between the radiographic image and the 3D reference object may be needed to perform the computation. It is also contemplated that shadows in the radiographic image may provide additional information about the relative positions of reference objects 118a, 118b, aiding in the accuracy of calibration. This may involve examining variations in shadow positions and shapes, and hence, can be used to further refine orientation and translation parameters.

Once the reference columns 112a-d have been verified for collinearity, distinctness, and for accuracy, they may then be used to establish correspondence between the 2D coordinates of the image and the 3D coordinates of reference device 110 and the other items in the radiographic image, such as fixation frame 10. In this regard, 3D coordinates for the reference objects 118a, 118b within reference device 110 should be known. Additionally, the coordinates of such reference objects 118a, 118b should be known relative to ring 20, the dimensions of which may also be known. Thus, the 3D coordinates for reference assembly 110, fixation frame 10, and bone may be mapped to the 2D radiographic image based on the above assessment of reference device 110.

At block 456, once the correspondence between 3D and 2D coordinates is established, pose estimations may be determined, and pose transformations on the 2D image may be performed. This may be achieved by applying a machine vision or camera calibration algorithm, such as the Tsai-Lenz calibration algorithm. Such algorithm may be used to determine a translational vector and rotation matrix that may be applied to image to effectively rotate and translate the radiographic image to take into account distortions and/or errors that may arise due to the perspective at which the radiographic image is taken of the 3D fixation frame 10, reference device 110, and bone.

At block 457, processor 410 may then estimate fixation frame parameters in terms of relative pose and position of rings 20 and 30 with respect to the bone and predefined anatomical features thereof. In this regard, processor 410 may make certain predefined measurements, such as ring offset measurements. Such measurements may then be used by processor 410 to generate a correction plan, as described in further detail in the aforementioned '150 Patent.

Although the subject matter disclosed herein has been described with reference to particular embodiments, it is to be understood that these embodiments are merely illustrative of the principles and applications exemplified by such embodiments. It is therefore to be understood that numerous modifications may be made to the exemplary embodiments and that other arrangements may be devised such as combining one or more features of one embodiment with another embodiment or features from a plurality of embodiments, as an example. Thus, the exemplary embodiments herein are not intended to be exhaustive or to limit the disclosed subject matter to those embodiments disclosed.

# EP 4 599 779 A1

**Claims**

1. A reference assembly connectable to a support ring of an external fixation frame, comprising:

   a mount device having a mount body, a static member, and a dynamic member, the static member being fixedly secured to and extending from the mount body and being configured to be received within a first hole of the support ring of the external fixation frame, the dynamic member being slidably connected to the mount body and being configured to be received within a second hole of the support ring; and
   a reference device connectable to the mount body, the reference device having a reference body and a plurality of reference objects disposed within the reference body, the reference objects each being made from a radiopaque material, and the reference body being made from a radiolucent material.

2. The reference assembly of claim 1, wherein the reference objects include a first reference object having a first cross-sectional dimension, and a second reference object having a second cross-sectional dimension, the first cross-sectional dimension being greater than the second cross-sectional dimension.

3. The reference assembly as in claim 1 or 2, wherein the reference objects are each spherical.

4. The reference assembly of claim 1, wherein the reference body includes a base and a plurality of reference columns extending from the base, each reference column including at least a first reference object and a second reference object, the first reference object having a larger cross-sectional dimension than the second reference object.

5. The reference assembly of claim 4, wherein the first reference object of each reference column is positioned further from the base than the second reference object.

6. The reference assembly as in one of claims 4 and 5, wherein each reference column includes a third and fourth reference object, the third and fourth reference objects each having a cross-sectional dimension equal to the cross-sectional dimension of the second reference object.

7. The reference assembly of claim 6, wherein the first, second, third, and fourth reference objects of each reference column are spherical.

8. The reference assembly as in one of claims 4-7, wherein the reference body includes four reference columns.

9. The reference assembly as in any one of claims 4-8, wherein the reference objects of each reference column are arranged colinearly.

10. The reference assembly as in any one of claims 4-9, wherein the first reference objects of the reference columns are coplanar.

11. The reference assembly of claim 6 or 7, or of any one of claims 8 to 10 when depending on claim 6 or 7, wherein the fourth reference objects of the reference columns are coplanar.

12. The reference assembly as in any one of claims 4-11, wherein the second reference objects of the reference columns are longitudinally staggered relative to one another.

13. The reference assembly as in any one of claims 4-12, wherein each reference column includes a longitudinal opening and a reference insert removably disposed therein, the reference insert including the first and second reference objects.

14. The reference assembly of claim 13, wherein the longitudinal opening of each reference column includes a threaded portion, and each reference column includes a threaded portion correspondingly engageable with the threaded portion of the longitudinal opening of the respective reference column.

15. The reference assembly as in any one of the preceding claims, wherein the mount body includes a slot, and the dynamic member is slidably disposed within the slot.

FIG. 1

FIG. 2

FIG. 3A

110

122c    116c    116a    116b

116d

118a
117a

118b

118b

122d

118b

117b

128

114

112

122b

122a

**FIG. 3B**

110

122d    122a

L    F    R

122c

112

D3

D1    D2

**FIG. 3C**

FIG. 3D

FIG. 4A

FIG. 4B

FIG. 5A

FIG. 5B

FIG. 5C

FIG. 5D

240

282

244

261    260

270

250

283

280

242

282

272

294        287

298        287

FIG. 6A

244

270        275

292

290

242        271

260        284

273

282        285

280        281

294        296

298        287

286

FIG. 6B

FIG. 7

Computing Device 400

Processor(s) 410

Memory 420

Instructions 422

Data 424

I/O 430

FIG.8

Mount Reference Assembly To Ring of Fixation Frame — 450

Obtain M-L and/or A-P Radiographic Images and Input Into Computational System — 451

Detect Reference Objects and Determine Reference Object Size and 2D Coordinates — 452

Identify and Verify Collinearity of Reference Objects in Discrete Reference Columns — 453

Establish Correspondence Between 3D and 2D Coordinates of Reference Objects — 454

Pose Estimation and Transformation — 456

Fixation Frame Parameter Estimation — 457

FIG. 9

Europäisches
Patentamt

European
Patent Office

Office européen
des brevets

# EUROPEAN SEARCH REPORT

Application Number

EP 25 15 5952

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/085183 A1 (ROSS JOHN D [US] ET AL) 29 March 2018 (2018-03-29) | 1-3,15 | INV. A61B17/62 |
| A | * figure 6a * | 4-14 | A61B17/64 A61B90/00 |
| | ----- | | |
| A | US 2023/086184 A1 (NOBLETT ANDREW P [US] ET AL) 23 March 2023 (2023-03-23) * figure 5G * | 1-15 | |
| | ----- | | |

**TECHNICAL FIELDS SEARCHED (IPC)**

A61B

The present search report has been drawn up for all claims

| Place of search | Date of completion of the search | Examiner |
|---|---|---|
| Munich | 11 April 2025 | Fernández Arillo, J |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 ...........................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 15 5952

This annex lists the patent family members relating to the patent documents cited in  the above-mentioned European search report.
The members are as contained in the European Patent Office EDP file on
The European Patent Office is in no way liable for these particulars which are merely given for the purpose of information.

11-04-2025

| Patent document cited in search report | Publication date | Patent family member(s) | Publication date |
|---|---|---|---|
| US 2018085183 A1 | 29-03-2018 | BR 112019005802 A2 | 02-07-2019 |
| | | EP 3515339 A1 | 31-07-2019 |
| | | US 2018085183 A1 | 29-03-2018 |
| | | WO 2018058140 A1 | 29-03-2018 |
| | | ZA 201901477 B | 30-09-2020 |
| US 2023086184 A1 | 23-03-2023 | AU 2021228690 A1 | 01-09-2022 |
| | | CN 115038401 A | 09-09-2022 |
| | | EP 4110219 A1 | 04-01-2023 |
| | | US 2023086184 A1 | 23-03-2023 |
| | | WO 2021173931 A1 | 02-09-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 16078023

- US 8654150 B